# EUROPEAN PATENT APPLICATION

(11) **EP 4 431 591 A2**
(43) Date of publication of application: **18.09.2024**
(21) Application number: 24460012.8
(22) Date of filing: 28.02.2024
(51) Int. Cl.: C12M 1/107, C12M 1/12, C12M 1/00

(54) **FLOW-THROUGH BIOGAS REACTOR**

(30) Priority: 17.03.2023 PL 44411023
(71) Applicant: Duda, Ludomir, 05-506 Magdalenka (PL)
(72) Inventor: Duda, Ludomir, 05-506 Magdalenka (PL)

(57) **Abstract**

The subject of the invention is a flow-through biogas reactor applicable to composting and biomass gasification processes. The flow-through biogas reactor, consisting of a set of parallel tubes, is characterized by the fact that it is equipped with a vertically connected chamber, parallel tubes placed one above the other are inclined from the horizontal by an angle α not less than 1 degree and closed unilaterally with clamps.

## Description

The subject of the invention is a flow-through biogas reactor applicable to biomass gasification processes. Various reactors for wet biomethane fermentation are commonly known, such as vertical tanks in the form of cylinders with diameters greater than their heights, and reactors in the form of horizontal tubes with diameters significantly smaller than their lengths, into which substrate in the form of biodegradable waste and silages is introduced. The volume of these tanks depends on the designed retention time and is a multiple of the daily substrate portions. In these tanks, substrate mixing with post-ferment is achieved using various types of dynamic mixers. However, such solutions are disadvantageous for the multi-stage methane fermentation process. Firstly, a significant portion of unfermented substrate is always present in the post-ferment, and secondly, this fermentation method is suboptimal for the biology of the multi-stage methane fermentation process. Consequently, disturbances caused by mixing components of different phases of the process lead to lower biogas production and higher organic dry matter content in the post-ferment.

From patent literature, the international invention WO2021089918A1 discloses a horizontal reactor equipped with a rotating shaft with blades for dispersing substrate matter, and biogas is collected from above the free surface of the upper part of the reactor tube. On the other hand, another international invention WO2013178621 A1 discloses a tubular reactor made of plastic, where the problem of foam reduction is solved by a system of foam quenching baffles. Meanwhile, from the German patent description DE102010010420A1, a reactor is known where the foam formation problem is solved by a system of semi-permeable baffles made in pockets for collecting biogas.

Also noteworthy is the solution according to the European patent EP2589429A1, which includes a laboratory reactor for biogas production containing a chamber for storing organic material during its fermentation with a mixer.

In another American invention US4670140A, a fixed bed of media is proposed in the reactor column for anaerobic decomposition processes. The column is divided by baffles into segments one above the other with intermediate spaces for gas removal from the column through discharge funnels to one or more suitable ascending tubes.

The solution according to the invention is a simpler, cheaper, and more efficient way of biogas production in all aspects by eliminating the shortcomings of previously known solutions.

The aim of the solution according to the invention is to ensure maximum biogas productivity while minimizing the biomass retention period in the reactor, by pre-mixing shredded and heated substrate with post-ferment. Further objectives of the solution according to the invention include reducing the risk of foam formation at the liquid-gas interface through the method of gas transport and reducing the free surface area of the liquid and gas phase interface, heavy metal separation in the form of sulfide sediment from post-ferment, and increasing the biomass reduction degree through post-ferment recirculation.

The flow-through biogas reactor [1], consisting of a set of parallel tubes, is characterized by the fact that it is equipped with a vertically connected chamber [5], parallel tubes [1, 2] placed one above the other are inclined from the horizontal by an angle α not less than 1 degree and closed unilaterally with clamps [3, 4].

Advantageously, the chamber [5] is terminated with a chimney [6] at the upper part and transitions into a funnel [7] with a discharge valve [8] at the lower part.

Advantageously, Archimedes' screws [9, 10] axially driven by motors [20,21] are installed in the parallel tubes [1, 2].

Advantageously, the Archimedes' screws [9, 10] are made in parallel tubes [1, 2] up to the edge of their connections with the chamber [5] at most.

Advantageously, the upper spigot [11] is located in the declination and equipped with a static mixer [12] in the form of alternating baffles or bolts made in the pipe conduit.

Advantageously, the lower spigot [13] is located in the declination.

Advantageously, the upper spigot [11] with the static mixer [12] and the lower spigot [13] are connected respectively by means of their upper and lower tees [19, 14] to the pipe conduit with the pump [15].

Advantageously, the upper spigot [11] with the static mixer [12] is connected to the pipe conduit with the pump [18].

Advantageously, the lower tee [14] is connected to the pipe conduit with a valve [16] connected to the level sensor [17] in the chamber [5].

Advantageously, the level sensor [17] is mounted in the chamber [5] above the upper edge of the parallel tube [1].

The parallel tubes [1, 2] inclined from the horizontal by an angle α not greater than 30 degrees are also advantageous.

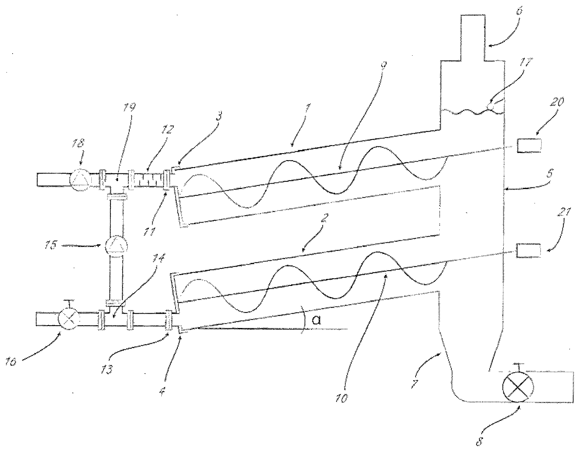

The invention has been exemplified in the accompanying drawing, which shows a schematic of the reactor in a side view.

## Claims

1. Flow-through biogas reactor constructed with at least two parallel pipes (1, 2), **characterized by** the fact that pipes (1, 2) inclined at an angle α not less than 1° are advantageous if arranged one above the other and closed on one side with covers (3, 4) respectively and connected on the other side by a chamber (5) ensuring the flow of substrate from pipe (1) to pipe (2).

2. The solution according to claim 1 is **characterized by** the fact that the chamber (5) is terminated at the top by a chimney (6) for biogas removal and at the bottom by a funnel (7) and valve (8) enabling the accumulation and removal of heavy substrate fractions.

3. The solution according to claim 1 is **characterized by** the fact that in pipes (1, 2) there are belts in the shape of Archimedes' screws (9, 10) mounted on shafts mounted in the axes of the pipes with drives (20, 21) moving sedimenting substrate fractions towards the edge of the pipe (1, 2) at their junction with the chamber (5).

4. The solution according to claim 1 is **characterized by** the fact that a fitting (11) is located in the cover (3) through which the mixture of substrate and post-ferment is pumped into pipe (1).

5. The solution according to claim 4 is **characterized by** the fact that a static mixer (12) is located on the extension of the fitting (11).

6. The solution according to claim 1 is **characterized by** the fact that a fitting (13) is located in the lower part of the cover (4).

7. The solution according to claims 5 and 6 is **characterized by** the fact that tees (14, 19) are connected by a pipe to a pump (15).

8. The solution according to claim 7 is **characterized by** the fact that the tee (14) is connected to a valve (16) whose opening degree is controlled by a signal from a substrate level sensor (17) in the upper part of the chamber (5).
